# FASCICULE DE BREVET EUROPEEN

(11) **EP 2 863 818 B1**
(45) Date de publication et mention de la délivrance du brevet: **31.01.2018**
(21) Numéro de dépôt: 13744621.7
(22) Date de dépôt: 25.06.2013
(51) Int. Cl.: A61B 17/86

(54) **VIS D'OSTEOSYNTHESE A COMPRESSION RADIALE REDUITE**
OSTEOSYNTHESESCHRAUBE MIT REDUZIERTER RADIALER KOMPRESSION
OSTEOSYNTHESIS SCREW WITH REDUCED RADIAL COMPRESSION

(30) Priorité: 26.06.2012 FR 1256066
(43) Date de publication de la demande: 29.04.2015
(73) Titulaire: In2Bones, 69130 Ecully (FR)
(72) Inventeur: GAUNEAU, Bertrand, Xavier, François, 69570 Dardilly (FR); FOURCAULT, Éric, Stéphane, 69370 Saint Didier au Mont d'Or (FR); GIET, Jean-Christophe, Alain, 69370 Saint Didier au Mont d'Or (FR)
(74) Mandataire: Weber, Jean-François
(86) Numéro de dépôt international: PCT/FR2013/051476
(87) Numéro de publication internationale: WO 2014/001705

(56) Documents cités:
- EP-A2- 1 767 160
- WO-A1-97/05830
- WO-A1-2008/156425
- WO-A2-2010/099239

## Description

### DOMAINE TECHNIQUE

La présente invention se rapporte au domaine technique de la chirurgie osseuse, et en particulier aux vis d'ostéosynthèse en général, de compression ou non compressives destinées à assurer la compression ou simplement stabiliser ou neutraliser la mise en place d'os sans assurer de compression des os.

La présente invention concerne une vis d'ostéosynthèse en général, qu'elle soit de compression ou non compressive formée d'une âme pourvue extérieurement d'un filet hélicoïdal, ladite vis comprenant une partie distale de pénétration et une partie proximale formant la tête de vis et ayant une section transversale conique.

### TECHNIQUE ANTERIEURE

Il est déjà connu d'avoir recours à des vis d'ostéosynthèse conçues pour ne pas assurer de compression des masses osseuses dans lesquelles elles sont vissées contrairement aux vis classiques d'ostéosynthèse à compression avec lesquelles le chirurgien cherche précisément à assurer lors du vissage, le rapprochement progressif des parties osseuses fracturées pour assurer leur mise en compression.

Dans certaines situations chirurgicales, il s'avère en effet particulièrement intéressant de ne pas assurer de compression des parties osseuses dont il convient d'assurer l'ostéosynthèse, une simple stabilisation, neutralisation, ou maintien en place des fractions osseuses s'avérant au contraire recherchée.

Ainsi, les situations chirurgicales les plus fréquentes impliquent, une fois que le chirurgien a défini les orientations des fractions osseuses à réparer ainsi que les directions de vissage, la mise en place d'une première vis de compression. Cette dernière est mise en place en vue d'obtenir une parfaite ostéo-intégration des fractions osseuses ce qui permet d'assurer une bonne ostéosynthèse.

Dans de telles situations, il s'avère particulièrement intéressant, pour éviter que l'ensemble des éléments ne risque de se déplacer relativement avant la fusion osseuse ou en cours de fusion osseuse, de mettre en place une seconde vis ayant pour seul objectif de neutraliser tout déplacement relatif des éléments entre eux après leur mise en compression.

Les vis d'ostéosynthèse qui permettent d'assumer une telle fonction sont des vis d'ostéosynthèse dites « *non compressives* » dans la mesure où elles sont spécifiquement conçues pour ne pas assurer de compression des fractions d'os qu'elles traversent lors du vissage.

En règle générale, les vis d'ostéosynthèse non compressives connues ont un profil de révolution cylindrique et sont pourvues d'un filetage hélicoïdal s'étendant sur toute la longueur de la vis.

Par ailleurs, et pour permettre d'obtenir suffisamment de matière et de résistance mécanique au niveau de l'empreinte du vissage, le profil de telles vis est conique.

Un tel profil permet en outre au chirurgien de mieux contrôler la pénétration de la vis d'ostéosynthèse dans la mesure où le couple de vissage devient de plus en plus important en cours de vissage à cause du profil conique de telles vis.

Cet avantage bien connu s'accompagne néanmoins d'un inconvénient en raison précisément de l'augmentation du couple de serrage lié à la forme conique, en particulier de la tête. Cette dernière est en effet susceptible de générer l'apparition de fissures au niveau de la masse osseuse en raison de l'existence d'un important effort de compression radiale.

Ce phénomène bien connu se traduit par la création de traits de refend.

On connait également des vis de compression qui sont conçues pour assurer la mise en compression de fragments osseux afin de réaliser leur ostéosynthèse. Ces vis peuvent posséder une tête conique laquelle est alors susceptible de générer, comme pour les vis d'ostéosynthèse non compressives, l'apparition de traits de refend.

On connaît du document EP-1 767 160 une vis d'ostéosynthèse formée d'une âme pourvue extérieurement d'un filet hélicoïdal, ladite vis comprenant une partie distale de pénétration et une partie proximale formant la tête de vis et comportant des encoches.

### EXPOSE DE l'INVENTION

La présente invention se propose par conséquent de porter remède aux différents inconvénients énumérés précédemment et de fournir une nouvelle vis d'ostéosynthèse permettant de réduire la compression radiale de la vis lors de son vissage tout en limitant le retrait de masse osseuse sans pour autant fragiliser la vis.

Un autre objet de l'invention vise à proposer une vis d'ostéosynthèse qui soit particulièrement efficace et robuste.

Un autre objet de l'invention vise à proposer une nouvelle vis d'ostéosynthèse qui soit particulièrement simple à fabriquer.

Un autre objet de l'invention vise à proposer une nouvelle vis d'ostéosynthèse dont la pénétration soit particulièrement facile.

Un autre objet de l'invention vise à proposer une nouvelle vis d'ostéosynthèse qui puisse être mise en place de manière particulièrement simple et rapide.

Les objets assignés à l'invention sont atteints à l'aide d'une vis d'ostéosynthèse formée d'une âme pourvue extérieurement d'un filet hélicoïdal, ladite vis comprenant une partie distale de pénétration et une partie proximale formant la tête de vis et ayant une section longitudinale conique caractérisée en ce que la partie proximale comporte des encoches circulaires qui sont ménagées dans l'épaisseur de l'âme et dans les intervalles inter filets de ladite partie proximale de manière à réduire la pression radiale lors du vissage.

### DESCRIPTIF SOMMAIRE DES DESSINS

D'autres particularités et avantages de l'invention apparaîtront et ressortiront plus en détails à la lecture de la description faite ci-après, en référence aux dessins annexés, donnés uniquement à titre d'exemple illustratif et non limitatif, dans lesquels :
- La figure 1 illustre, selon une vue de côté, une vis d'ostéosynthèse non compressive conforme à l'invention.
- La figure 2 illustre, selon une vue partielle agrandie de la figure 1, un détail de réalisation d'une vis d'ostéosynthèse non compressive conforme à l'invention.
- La figure 3 illustre, selon une vue en perspective partielle, un exemple de réalisation d'une vis d'ostéosynthèse non compressive conforme à l'invention.
- La figure 4 illustre, selon une vue de dessus, un exemple de réalisation d'une vis d'ostéosynthèse non compressive conforme à l'invention.

### MEILLEURE MANIERE DE REALISER L'INVENTION

Dans la description et les dessins qui suivent, il sera fait référence à une vis d'ostéosynthèse non compressive à titre d'exemple purement illustratif et non limitatif étant entendu que l'invention peut bien évidemment être appliquée à une vis d'ostéosynthèse à compression de telle sorte que l'invention concerne une vis d'ostéosynthèse en général qu'elle soit compressive ou non compressive.

A titre préférentiel, la vis selon l'invention est constituée d'une vis d'ostéosynthèse non compressive.

La vis d'ostéosynthèse non compressive 1 illustrée aux figures se présente sous la forme générale d'une vis oblongue de forme générale sensiblement cylindrique présentant un axe d'extension longitudinal X-X' définissant également l'axe autour duquel la vis sera mise en rotation afin de pénétrer dans les masses osseuses.

La vis d'ostéosynthèse selon l'invention est de préférence une vis d'ostéosynthèse non compressive 1 c'est-à-dire une vis conçue pour ne pas générer d'effort de compression axiale susceptible d'assurer le rapprochement puis la mise en compression de fragments osseux que la vis sera amenée à traverser.

En ce sens, la vis d'ostéosynthèse non compressive 1 selon l'invention est par conséquent et de préférence une vis conçue et destinée uniquement à assurer, sécuriser ou neutraliser la position des fragments osseux qui ont été préalablement mis en compression par le biais d'une vis d'ostéosynthèse de compression classique.

Ainsi, la vis d'ostéosynthèse non compressive 1 conforme à l'invention et décrite ci-après est une vis conçue et destinée à éviter tout déplacement relatif non souhaité entre les masses et fragments osseux dans lesquels la vis d'ostéosynthèse non compressive 1 conforme à l'invention est vissée.

Tel qu'illustré aux figures, la vis d'ostéosynthèse non compressive 1 conforme à l'invention est formée d'une âme 2 centrale se présentant de manière générale sous la forme d'un corps de révolution sensiblement cylindrique d'axe longitudinal X-X' et de section constante ou non, ladite âme 2 étant creuse ou pleine.

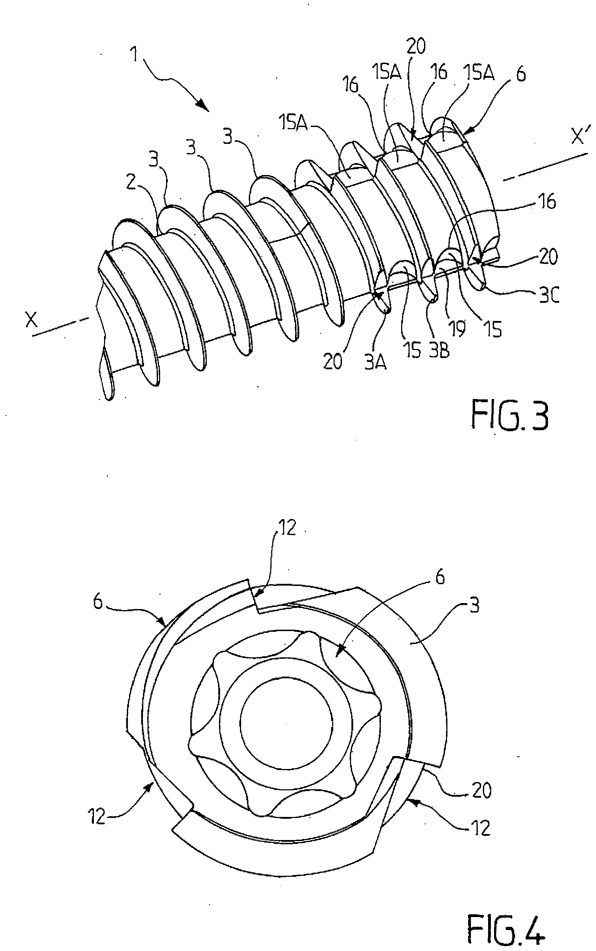

Dans le cas où l'âme 2 est de section creuse, la vis d'ostéosynthèse non compressive 1 selon l'invention est donc une vis canulée tel qu'illustré à la figure 4.

Selon l'invention, la vis d'ostéosynthèse non compressive 1 est formée de l'âme 2 qui est pourvue extérieurement d'un filet hélicoïdal 3, ladite vis comprenant une partie centrale 11 située entre une partie distale de pénétration 4 et une partie proximale 5 formant la tête de vis 6 et ayant une section longitudinale conique lui conférant donc un profil conique ou sensiblement conique.

Le filet hélicoïdal 3, de pas constant ou non, définit en considération de l'axe X-X', des intervalles inter filets 13 de pas correspondant à la surface périphérique de l'âme 2.

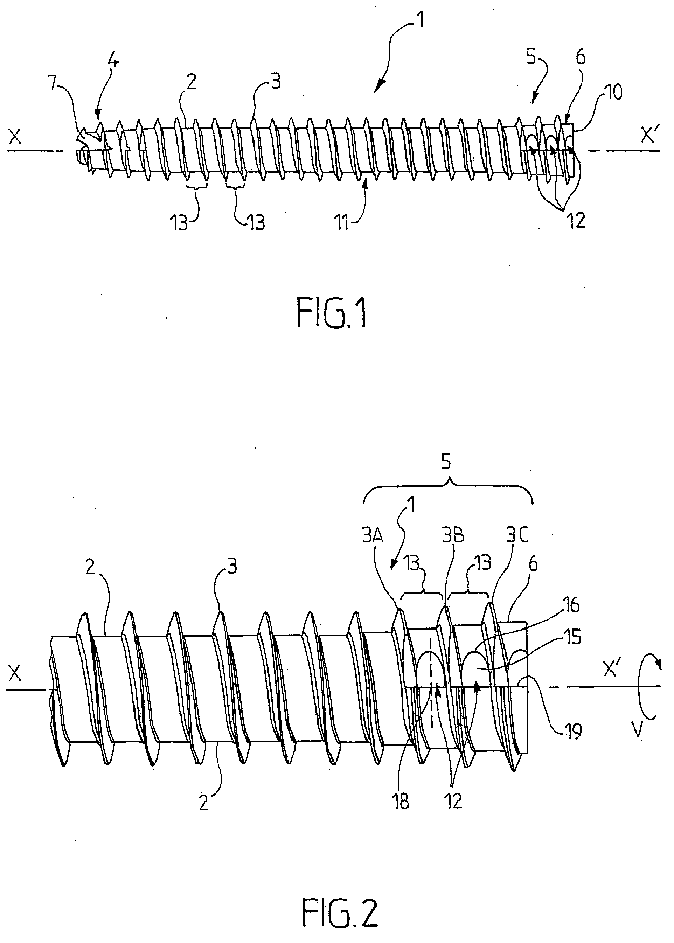

Selon l'invention, la partie distale de pénétration 4 est de préférence auto-taraudante telle qu'illustré à la figure 1 et est pourvue à cet effet de fentes 7 et des découpes appropriées.

De préférence, la partie distale de pénétration 4 présente un diamètre plus faible à l'extrémité proximale de la vis d'ostéosynthèse 1 qu'à proximité de la partie centrale 11, afin d'améliorer et de faciliter l'action auto-perforante et / ou auto-taraudante de ladite vis d'ostéosynthèse 1 lors du vissage de celle-ci.

Selon l'invention, la tête de vis 6 peut être réalisée de manière classique sous la forme d'une tête 6 « *enfouie* » dont le diamètre de la section augmente progressivement à partir de l'âme 2 et de la fin de la partie centrale 11 jusqu'à l'extrémité terminale 10 de la partie proximale 5.

Avantageusement, le filet hélicoïdal 3 s'étend sur toute la longueur de la vis de telle sorte que l'âme 2 est recouverte du filet hélicoïdal 3 à partir de l'extrémité 10 de la tête de vis 6 jusqu'à l'extrémité de la partie distale 4 y compris la partie centrale 11 de la vis.

A titre de variante (non représentée aux figures), le filet hélicoïdal 3 peut néanmoins ne pas s'étendre sur la totalité de la vis, et être par exemple réalisé en deux tronçons, la partie centrale 11 étant dépourvue de filets.

La présence d'une tête de vis 6 avec une section longitudinale conique dont le diamètre augmente vers l'extrémité 10 et qui est de plus large diamètre que le reste de la vis, permet d'obtenir une bonne résistance lors du vissage ce qui permet au chirurgien de contrôler la pénétration de la vis en raison de l'augmentation du couple lorsque la tête de vis 6 pénètre dans la masse osseuse.

De préférence, la tête de vis 6 est de forme générale tronconique, de sorte que son diamètre est le plus élevé à proximité de l'extrémité terminale 10 et est sensiblement égal à celui de la fin de la partie centrale 11 au niveau de l'autre extrémité de ladite tête de vis 6.

Selon une particularité importante de l'invention, la partie proximale 5 comporte des encoches circulaires 12 qui sont ménagées dans l'épaisseur de l'âme 2 et dans les intervalles inter filets 13 de ladite partie proximale 5 de manière à réduire la pression radiale lors du vissage.

Ainsi, selon l'invention, la présence d'encoches circulaires 12 s'étendant donc dans une direction correspondant à la rotation de la vis et ménagées dans la partie de plus fort diamètre de la vis, savoir au niveau de la tête de vis 6, évite la création d'une pression radiale trop importante à ce niveau puisque le diamètre de l'âme est partiellement et ponctuellement réduit dans cette zone en raison des enlèvements de matière créant des zones de dépression résultant des encoches 12. Cette particularité constructive permet ainsi d'éviter la création de traits de refend dans la masse osseuse traversée tout en évitant de trop fragiliser la vis qui reste par ailleurs bien équilibrée géométriquement lors de sa pénétration dans les masses osseuses.

Sous l'expression « *encoches circulaires* » on entend ainsi au sens de l'invention, des encoches qui forment des cavités dans la surface de l'âme 2 dans la zone située entre deux filets consécutifs du filet hélicoïdal 3 et qui s'étendent de manière générale dans le sens circulaire en considération de la section circulaire de l'âme 2, par opposition aux encoches longitudinales qui s'étendent de manière générale dans le sens longitudinal de la vis en considération de son axe longitudinal X-X'.

Ainsi, les encoches circulaires 12 forment des cavités ponctuelles, lesquelles sont ménagées dans l'épaisseur de l'âme 2 et disposées à partir de la périphérie de ladite âme 2, et dont l'ouverture est orientée ortho-radialement à l'axe d'extension longitudinale X-X' de la vis d'osthéosynthèse 1 (c'est-à-dire orientée de façon perpendiculaire à un rayon de l'axe d'extension longitudinale X-X').

Tel qu'illustré aux figures 3 et 4, la vis d'ostéosynthèse 1 selon l'invention comporte plusieurs encoches circulaires 12 alignées axialement les unes au-dessus des autres dans des intervalles inter filet 13 consécutifs pour former au moins une rangée d'encoches 12.

Selon l'invention, les encoches 12 sont de préférence ménagées dans l'intervalle inter filet 13 créé entre deux portions de filets adjacents 3A, 3B, 3C ... par le filet hélicoïdal 3. On obtient ainsi des encoches circulaires 12 qui sont régulièrement alignées axialement les unes au-dessus des autres de telle sorte que la réduction de compression radiale obtenue est régulière et bien équilibrée.

Tel qu'illustré par exemple à la figure 3, une rangée d'encoches 12 sera formée par plusieurs cuvettes 15A alignées axialement les unes au-dessus des autres dans des intervalles inter filet 13 consécutifs ou adjacents.

A titre de variante complémentaire, tel qu'illustré par exemple à la figure 3, la vis d'ostéosynthèse non compressive 1 selon l'invention comporte plusieurs rangées d'encoches 12 réparties angulairement à intervalles réguliers autour de la périphérie de l'âme 2.

Selon une version particulièrement avantageuse de l'invention, les encoches 12 forment une cuvette 15 ayant une courbure concave (figure 3) définissant un bord d'attaque aval 16, de préférence ovale, en considération du sens de vissage V.

De préférence, le bord d'attaque aval 16 est une arête saillante formée par l'intersection géométrique de la cuvette 15 et de l'âme 2, ladite arête présentant par exemple la forme d'une portion de courbe bicylindrique, ou d'une portion d'ellipse dont le grand rayon est sensiblement orienté en direction du sens de vissage V.

Ainsi, selon l'invention, le bord d'attaque aval 16 est ouvert ce qui facilite la pénétration dans la masse osseuse lors du vissage selon le sens V.

Tel qu'illustré, la cuvette 15 est de forme allongée et présente un axe longitudinal 18 qui s'étend dans un plan médian sensiblement parallèle et équidistant au plan d'extension de deux portions de filets consécutives 3A, 3B (figure 2).

De manière particulièrement avantageuse, la cuvette 15 est pourvue d'un bord amont 19 qui est fermé formant une cloison venant fermer la cuvette 15.

Tel qu'illustré à la figure 3 en particulier, le bord amont 19 forme une cloison qui est orthogonale à la surface périphérique de l'âme 2. Toutes ces caractéristiques confèrent ainsi à la vis selon l'invention une bonne stabilité lors du vissage en raison du bon équilibrage des forces générées ainsi qu'une pression radiale réduite voir inexistante.

A titre purement illustratif, la vis d'ostéosynthèse non compressive 1 selon l'invention pourra comporter deux voire trois rangées d'encoches circulaires 12, voire plus. Dans le cas où la vis selon l'invention comporte deux rangées d'encoches circulaires 12, les rangées seront décalées angulairement de 180 degrés, et dans le cas où la vis selon l'invention comporte trois rangées d'encoches circulaires 12, ces dernières seront angulairement décalées de 120 degrés.

A titre de variante complémentaire, tel qu'illustré aux figures, les encoches circulaires 12 sont associées à des découpes 20 d'auto taraudage ménagées dans l'épaisseur du filet hélicoïdal (3). Les découpes 20 sont ménagées dans l'épaisseur même du filet hélicoïdal 3 et en continuité axiale avec les encoches circulaires 12 associées et adjacentes.

Cette variante de réalisation préférentielle est illustrée aux figures et en particulier à la figure 4 qui montre la présence de trois rangées d'encoches circulaires 12, décalées angulairement de 120 degrés, les encoches circulaires 12 étant associées à des découpes 20 en forme de L, de préférence à angle droit ménagées, lesdites découpes 20 étant taillées dans l'épaisseur d'une portion de filet 3 au droit des encoches circulaires 12.

De préférence, chacune des découpes 12 sont ménagées dans l'épaisseur d'une portion de filet 3 afin de présenter une première arête de découpe orientée de façon sensiblement ortho-radiale à l'axe d'extension longitudinale X-X', et une deuxième arête de découpe orientée de façon sensiblement radiale, ou sensiblement parallèle à un rayon de l'axe d'extension longitudinale X-X', de sorte que les première et deuxième arêtes de découpe forment un L dont les extrémités se situent à la périphérie d'une portion de filet 3.

Néanmoins, au sens de l'invention, les encoches 12 peuvent n'être ménagées que dans l'épaisseur de l'âme 2, aucune découpe 20 n'étant alors réalisée dans l'épaisseur du filet hélicoïdal 3 lequel peut donc s'étendre de la partie proximale 5 à la partie distale 4 sans aucune découpe 20.

La vis d'ostéosynthèse 1 selon l'invention est mise en place par le chirurgien après que ce dernier ait au préalable déjà mis en place une première vis de compression ayant pour but de rapprocher et mettre en compression les fragments osseux qu'il convient précisément de fusionner par ostéosynthèse.

La vis d'ostéosynthèse 1 selon l'invention est alors vissée dans la zone appropriée en vue de neutraliser tout déplacement éventuel des éléments osseux et de stabiliser les éléments mis en compression par le chirurgien.

Le vissage s'effectue de manière classique à l'aide d'un ancillaire de vissage (non représenté) inséré par exemple dans la tête de vis 6, la vis d'ostéosynthèse non compressive 1 pénétrant ensuite naturellement dans la masse osseuse grâce à la partie distale de pénétration 4 auto-taraudante. Les encoches circulaires 12 permettent d'éviter, lors de la rotation dans le sens V de la vis, la création d'une pression radiale trop importante, de préserver une part importante du capital osseux sans pour autant fragiliser la vis d'ostéosynthèse.

Par ailleurs, la répartition homogène des encoches 12 dans la masse de la vis et autour de cette dernière permet d'obtenir un bon équilibrage du vissage induisant une pénétration régulière et contrôlée de la vis d'ostéosynthèse non compressive 1 conforme à l'invention.

### POSSIBILITE D'APPLICATION INDUSTRIELLE

L'invention trouve son application industrielle dans le domaine technique de la chirurgie osseuse, et en particulier dans le domaine des vis d'ostéosynthèse.

## Revendications

1. - Vis d'ostéosynthèse (1) formée d'une âme (2) pourvue extérieurement d'un filet hélicoïdal (3), ladite vis comprenant une partie distale de pénétration (4) et une partie proximale (5) formant la tête de vis (6) et comportant des encoches (12), **caractérisée en ce que** la partie proximale (5) a une section longitudinale conique et **en ce que** lesdites encoches (12) sont circulaires, s'étendant de manière générale dans le sens circulaire en considération de la section circulaire de l'âme, et sont ménagées dans l'épaisseur de l'âme (2) et dans les intervalles inter filets (13) de ladite partie proximale (5) de manière à réduire la pression radiale lors du vissage.

2. - Vis d'ostéosynthèse (1) selon la revendication 1 **caractérisée en ce qu'**elle comporte plusieurs encoches circulaires (12) alignées axialement les unes au-dessus des autres dans les intervalles inter filet (13) pour former au moins une rangée d'encoches (12).

3. - Vis d'ostéosynthèse (1) selon la revendication 2 **caractérisée en ce que** les encoches circulaires (12) sont ménagées dans des intervalles inter filets (13) adjacents.

4. - Vis d'ostéosynthèse (1) selon la revendication 2 ou 3 **caractérisée en ce qu'**elle comporte plusieurs rangées d'encoches circulaires (12) réparties angulairement à intervalles réguliers autour de la périphérie de l'âme (2).

5. - Vis d'ostéosynthèse (1) selon l'une des revendications 1 à 4 **caractérisée en ce que** les encoches circulaires (12) forment une cuvette (15) ayant une courbure concave définissant un bord d'attaque aval (16) en considération du sens de vissage V.

6. - Vis d'ostéosynthèse (1) selon la revendication 5 **caractérisée en ce que** la cuvette (15) est pourvue d'un bord amont (19) fermé formant une cloison.

7. - Vis d'ostéosynthèse (1) selon l'une des revendications 1 à 6 **caractérisée en ce que** les encoches circulaires (12) sont associées à des découpes (20) d'auto taraudage ménagées dans l'épaisseur du filet hélicoïdal (3).

8. - Vis d'ostéosynthèse (1) selon l'une des revendications 1 à 7 **caractérisée en ce qu'**elle est canulée.

9. - Vis d'ostéosynthèse (1) selon l'une des revendications 1 à 8 **caractérisée en ce que** le filet hélicoïdal (3) s'étend sur toute la longueur de la vis.

10. - Vis d'ostéosynthèse (1) selon l'une des revendications 1 à 9 **caractérisée en ce que** la partie distale de pénétration (4) est auto-taraudante.

11. - Vis d'ostéosynthèse (1) selon l'une des revendications précédentes **caractérisée en ce qu'**elle est constituée par une vis non compressive.

## Patentansprüche

1. - Osteosyntheseschraube (1), die aus einem Kern (2) gebildet ist, der außen mit einem Spiralgewinde (3) versehen ist, wobei die Schraube einen distalen Eindringteil (4) und einen proximalen Teil (5) aufweist, der den Schraubenkopf (6) bildet und Kerben (12) aufweist, **dadurch gekennzeichnet, dass** der proximale Teil (5) einen konischen Längsschnitt aufweist und dass die Kerben (12) kreisförmig sind und sich dabei im Allgemeinen im Sinne des kreisförmigen Querschnitts des Kerns in der kreisförmigen Richtung erstrecken und in der Dicke des Kerns (2) und in den Gewindezwischenräumen (13) des proximalen Teils (5) derart ausgebildet sind, um den radialen Druck beim Einschrauben zu reduzieren.

2. - Osteosyntheseschraube (1) nach Anspruch 1, **dadurch gekennzeichnet, dass** sie mehrere kreisförmige Kerben (12) aufweist, die in den Gewindezwischenräumen (13) übereinander axial ausgerichtet sind, um mindestens eine Reihe von Kerben (12) zu bilden.

3. - Osteosyntheseschraube (1) nach Anspruch 2, **dadurch gekennzeichnet, dass** die kreisförmigen Kerben (12) in benachbarten Gewindezwischenräumen (13) angeordnet sind.

4. - Osteosyntheseschraube (1) nach Anspruch 2 oder 3, **dadurch gekennzeichnet, dass** sie mehrere Reihen von kreisförmigen Kerben (12) aufweist, die winkelmäßig in regelmäßigen Abständen um den Umfang des Kerns (2) verteilt sind.

5. - Osteosyntheseschraube (1) nach einem der Ansprüche 1 bis 4, **dadurch gekennzeichnet, dass** die kreisförmigen Kerben (12) eine Mulde (15) bilden, die eine konkave Krümmung aufweist, die im Sinne der Einschraubrichtung V einen stromabwärtigen Angriffsrand (16) definiert.

6. - Osteosyntheseschraube (1) nach Anspruch 5, **dadurch gekennzeichnet, dass** die Mulde (15) mit einem geschlossenen stromaufwärtigen Rand (19) versehen ist, der eine Trennwand bildet.

7. - Osteosyntheseschraube (1) nach einem der Ansprüche 1 bis 6, **dadurch gekennzeichnet, dass** die kreisförmigen Kerben (12) mit selbstschneidenden Ausschnitten (20) versehen sind, die in der Dicke des Spiralgewindes (3) ausgebildet sind.

8. - Osteosyntheseschraube (1) nach einem der Ansprüche 1 bis 7, **dadurch gekennzeichnet, dass** sie kanüliert ist.

9. - Osteosyntheseschraube (1) nach einem der Ansprüche 1 bis 8, **dadurch gekennzeichnet, dass** sich das Spiralgewinde (3) über die gesamte Länge der Schraube erstreckt.

10. - Osteosyntheseschraube (1) nach einem der Ansprüche 1 bis 9, **dadurch gekennzeichnet, dass** der distale Eindringteil (4) selbstschneidend ist.

11. - Osteosyntheseschraube (1) nach einem der vorhergehenden Ansprüche, **dadurch gekennzeichnet, dass** sie durch eine Schraube ohne Kompression gebildet ist.

## Claims

1. - An osteosynthesis screw (1) formed by a core (2) provided externally with a helical thread (3), said screw comprising a distal penetration portion (4) and a proximal portion (5) forming the screw head (6) and including notches (12), **characterised in that** the proximal portion (5) has a conical longitudinal section and **in that** said notches (12) are circular, extending generally in the circular direction relative to the circular section of the core, and are provided in the thickness of the core (2) and in the inter-thread gaps (13) of said proximal portion (5) so as to reduce radial pressure during screw tightening.

2. - An osteosynthesis screw (1) according to claim 1, **characterised in that** it includes a plurality of circular notches (12) axially aligned one after the other in the inter-thread gaps (13) so as to form at least one row of notches (12).

3. - An osteosynthesis screw (1) according to claim 2, **characterised in that** the circular notches (12) are provided in adjacent inter-thread gaps (13).

4. - An osteosynthesis screw (1) according to claim 2 or 3, **characterised in that** it includes a plurality of rows of circular notches (12) angularly distributed at regular intervals around the periphery of the core (2).

5. - An osteosynthesis screw (1) according to one of claims 1 to 4, **characterised in that** the circular notches (12) form a depression (15) of concave curvature, defining a leading edge (16) that is downstream relative to the screw-tightening direction V.

6. - An osteosynthesis screw (1) according to claim 5, **characterised in that** the depression (15) is provided with a closed upstream edge (19) forming a partition.

7. - An osteosynthesis screw (1) according to one of claims 1 to 6, **characterised in that** the circular notches (12) are associated with self-tapping cut-outs (20) provided in the thickness of the helical thread (3).

8. - An osteosynthesis screw (1) according to one of claims 1 to 7, **characterised in that** it has a cannula.

9. - An osteosynthesis screw (1) according to one of claims 1 to 8, **characterised in that** the helical thread (3) extends over the entire length of the screw.

10. - An osteosynthesis screw (1) according to one of claims 1 to 9, **characterised in that** the distal penetration portion (4) is self-tapping.

11. - An osteosynthesis screw (1) according to one of the preceding claims, **characterised in that** it is constituted by a non-compressive screw.
